# EUROPEAN PATENT SPECIFICATION

(11) **EP 4 058 004 B1**
(45) Date of publication and mention of the grant of the patent: **06.03.2024**
(21) Application number: 20811102.1
(22) Date of filing: 11.11.2020
(51) Int. Cl.: A61K 31/12, A61K 31/397, A61K 31/7052, A61P 35/00

(54) **EZETIMIBE AND CURCUMIN FOR USE IN CANCER TREATMENT**
EZETIMIB UND CURCUMIN ZUR VERWENDUNG BEI DER KREBSBEHANDLUNG
ÉZÉTIMIBE ET CURCUMINE POUR L'UTILISATION DANS LE TRAITEMENT DU CANCER

(30) Priority: 12.11.2019 ZA 201907474
(43) Date of publication of application: 21.09.2022
(73) Proprietor: University of South Africa, 0001 Pretoria (ZA)
(72) Inventor: NTWASA, Monde, 2193 Johannesburg (ZA)
(74) Representative: Sagittarius IP
(86) International application number: PCT/IB2020/060599
(87) International publication number: WO 2021/094933

(56) References cited:
- Charmy Starnod Twala: "DRUGS TARGETING THE RETINOBLASTOMA BINDING PROTEIN 6 (RBBP6)" In: "DRUGS TARGETING THE RETINOBLASTOMA BINDING PROTEIN 6 (RBBP6)", 3 November 2017 (2017-11-03), Faculty of Science, University of the Witwatersrand, Johannesburg, XP055769840, page 40, paragraph 4.4 - page 50 page 47, paragraph 4.4.4 page 48 page 60, paragraph 5.6 page 49 - page 50; figures 4.18,4.19
- KEITH R. SOLOMON ET AL: "Ezetimibe Is an Inhibitor of Tumor Angiogenesis", AMERICAN JOURNAL OF PATHOLOGY., vol. 174, no. 3, 1 March 2009 (2009-03-01) , pages 1017-1026, XP055769821, US ISSN: 0002-9440, DOI: 10.2353/ajpath.2009.080551
- KOUICHI MIURA ET AL: "Ezetimibe suppresses development of liver tumors by inhibiting angiogenesis in mice fed a high-fat diet", CANCER SCIENCE, vol. 110, no. 2, 1 February 2019 (2019-02-01), pages 771-783, XP055769819, JP ISSN: 1347-9032, DOI: 10.1111/cas.13902
- MAO LI ET AL: "Curcumin, a Dietary Component, Has Anticancer, Chemosensitization, and Radiosensitization Effects by Down-regulating the MDM2 Oncogene through the PI3K/mTOR/ETS2 Pathway", CANCER RESEARCH, vol. 67, no. 5, 1 March 2007 (2007-03-01), pages 1988-1996, XP055557074, US ISSN: 0008-5472, DOI: 10.1158/0008-5472.CAN-06-3066
- MALJAARS J ET AL: "990 Mechanisms of Growth Inhibition of Chemo-Surviving Colon Cancer Cells By Curcumin and Resveratrol", GASTROENTEROLOGY, ELSEVIER INC, US, vol. 134, no. 4, 1 April 2008 (2008-04-01) , pages A-148, XP023432483, ISSN: 0016-5085, DOI: 10.1016/S0016-5085(08)60686-1 [retrieved on 2008-04-01]

## Description

THIS INVENTION RELATES TO a pharmaceutical composition comprising ezetimibe for use in the treatment of cancer. It relates in particular, to the pharmaceutical composition and ezetimibe for use in the treatment of cancers that overexpress Mdm2.

Ezetimibe is a United States Food and Drug Administration (FDA) approved drug which blocks cholesterol uptake in the intestines thereby reducing its concentration in the systemic circulation. It is thus used to treat hypercholesterolaemia. After oral administration, ezetimibe is rapidly glucuronidated and recycled by the enterohepatic circulation to its target site in the intestine. It is reported that this glucuronide metabolite is at least as potent as ezetimibe with respect to inhibition of cholesterol uptake. This is based on experiments using the initial drug lead, SCH48461 which was found to inhibit cholesterol absorption by 70% whereas the metabolite inhibited absorption by more than 95%. Furthermore, the metabolite was retained on the intestinal wall and the bulk of it was not systemically available.

Currently, the known target of ezetimibe is the *Niemann-Pick C1-Like 1* (NPC1L1) found in jejunal enterocytes. Up to 80% of ezetimibe is rapidly metabolised in the intestine to its pharmacologically active glucuronide metabolite (EZE-GLUC) by uridine 5-diphosphate (UDP)-glucuronosyl-transferase (UGT) 1A1, 1A3, and 2B15. The remaining parent drug and the glucuronidated metabolite are then excreted into the bile via the portal vein, and delivered back into the intestinal site of action, thereby increasing the half-life of the drug. Cholesterol absorption studies indicated that the glucuronide appeared more potent than ezetimibe itself because glucuronidated ezetimibe localises more avidly to the intestine. Ezetimibe and/or the glucuronide metabolite are excreted in the faeces (90%) and urine (10%).

Charmy Starnod Twala: "Drugs Targeting the Retinoblastoma Binging Protein 6 (RBBP6)" in "Drugs Targeting the Retinoblastoma Binging Protein 6 (RBBP6)", 3 November 2017 (2017-11-03), Faculty of Science, University of the Witwatersrand, Johannesburg, studies the three-dimensional structure of the RBBP6 domains with emphasis on cancer and special focus in the p53 domain and the interaction with Mdm2.

Keith R. Solomon et al: "Ezetimibe is an Inhibitor of Tumor Angiogenesis", American Journal of Pathology, vol. 174, no. 3, 1 March 2009 (2009-02-01), pages 1017-1026 studies ezetimibe in combination with hyper- and hypocholesterol diets, showing that elevated circulating cholesterol levels promote, whereas a reduction in circulating cholesterol levels retard, the growth of human prostate cancer xenograft tumors in mice.

Kouichi Miura et al.: "Ezetimibe suppresses development of liver tumors by inhibiting angiogenesis in mice fed a high-fat diet", Cancer Science, vol. 110, no. 2, 1 February 2019 (2019-02-01), pages 771-783 studies ezetimibe in regards to the prevention of NASH-related liver cirrhosis and HCC, concluding that ezetimibe suppressed development of liver tumors by inhibiting angiogenesis in *Pten^{Δhep}* mice with hypercholesterolemia.

Mao Li et al.: "Curcumin, a Dietary Component, has Anticancer Chemosensitization, and Radiosensitization Effects by Down-Regulating the MDM2 Oncogene through the PI3K/mTOR/ETS2 Pathway", Cancer Research, vol. 67, no.5, 1 March 2007 (2007-03-01), pages 1988-1996 identifies an studies curcumin as an inhibitor of MDM2 expression and studies its function as an anti-cancer agent.

Bhaumik B. Patel et al.: "990 Mechanism of Growth Inhibition of Chemo-Surviving Colon Cancer Cells by Curcumin and Resveratrol", Gastroenterology, Elsevier Inc, US, vol. 134, no. 4, 1 April 2008 (2008-04-01), pages A-148 discloses that curcumin and resveratrol are known to inhibit initiation, promotion and progression of carcinogenesis and studies whether addition of curcumin or resveratrol to FOLFOX will be a superior therapeutic strategy for chemo-surviving cells.

The Mouse Double minute protein (Mdm2) is the prototypical negative regulator of the tumour suppressor protein, p53 which is inactive under normal physiological conditions and is only activated when cells are under stress or certain cellular functions are impaired. Inactivation of the tumor suppressor p53 and/or overexpression of the oncogene Mdm2 frequently occur in human cancers, and are associated with poor prognosis, advanced forms of the disease, and chemoresistance. Anti-cancer drug development therefore seeks to target the interaction between Mdm2 and p53. Nutlins are spirooxindole-derived compounds whose mechanism of action is to disrupt this interaction and are under development and in clinical trials. Here, it is shown that ezetimibe is a candidate drug that may target this interaction and may have better drug-likeness than nutlins. Furthermore, when combined with a compound, such as curcumin, ezetimibe may be suitable for treatment of colon cancer cells since its conversion to EZE-GLUC would be prevented.

According to a first aspect of the invention, there is provided a pharmaceutical composition comprising ezetimibe, or a pharmaceutically acceptable salt thereof, and curcumin for use in a method of treating a cancer in a patient in need thereof, wherein the cancer is characterized by overexpression of Mdm2.

The cancer may be selected from a solid cancer, such as colon cancer, or a melanoma.

The pharmaceutical composition may further comprise a pharmaceutically acceptable excipient, carrier or diluent and may be formulated for systemic or oral administration to the patient.

The pharmaceutical composition may be in the form of a formulation, an injection, a tablet, a capsule, or other suitable form for administration.

According to an aspect of the disclosure, there is described a method of producing the pharmaceutical composition of the first aspect of the invention.

The pharmaceutical composition may be formulated for oral administration to the patient.

Ezetimibe, or a pharmaceutically acceptable salt thereof, may bind to the p53-binding domain of Mdm2 to promote the tumour suppressing activity of p53 and may induce apoptosis of cancer cells in the patient.

According to a second aspect of the invention, there is provided a pharmaceutical composition comprising ezetimibe, or a pharmaceutically acceptable salt thereof, for use in a method of treating a cancer in a patient in need thereof, wherein the pharmaceutical composition is for administration with curcumin and wherein the cancer is characterized by overexpression of Mdm2.

The cancer is characterized by an overexpression of Mdm2, and may be a solid cancer, including colon cancer, or a melanoma.

The pharmaceutical composition may be formulated for systemic or oral administration to the patient, and may be in the form of a formulation, an injection, a tablet, or a capsule.

The cancer may be selected from breast cancer, cervical cancer, colorectal cancer, fallopian tube carcinoma, gastric cancer, glioma, Hodgkin's lymphoma, leukemia, liver cancer, lung cancer, melanoma, non-Hodgkin's lymphoma, oral cancer, ovarian cancer, pancreatic cancer, renal cell carcinoma, thyroid cancer, and other cancers.

Ezetimibe, or a pharmaceutically acceptable salt thereof, may be formulated for systemic administration to the patient to introduce the ezetimibe, or pharmaceutically acceptable salt thereof, directly into the circulatory system.

Ezetimibe, or a pharmaceutically acceptable salt thereof, may be formulated for administration via infusion, implantation, transdermal or other means.

The ezetimibe, or a pharmaceutically acceptable salt thereof, and curcumin may be formulated for oral administration to the patient.

The invention will now be described in more detail with reference to the Example hereunder, and the accompanying drawings.

In the drawings
FIGURE 1 shows, for the Example, the molecular interaction of Mdm2 with ezetimibe (A and B), with nutlin3a (C and D), and a superimposition showing the interaction of Mdm2 with ezetimibe and nutlin3a (E and F);
FIGURE 2 shows, for the Example, toxicity assays using MTT assays with (A) A375 malignant melanoma cells treated with ezetimibe, (B) PANC-1 pancreatic cancer cells treated with ezetimibe and (C) HEK293 human embryonic kidney cells treated with ezetimibe;
FIGURE 3 shows, for the Example, the mechanism of action of curcumin; and
FIGURE 4 shows the structure of (A) ezetimibe and (B) ezetimibe phenoxy glucuronide.

### Material and Methods

### Cell lines

The human malignant melanoma (A375) and human embryonic kidney (HEK293) cell lines were donations from Dr Abimbola Arc from the University of Pretoria, South Africa and the pancreatic cancer (PANC-1) cell line was donated by Dr Ekene Nweke from the University of the Witwatersrand, South Africa.

### Media and chemicals

The following reagents were used: DMEM (Lonza Bioscience, USA); foetal bovine serum (FBS) (Biowest, USA); penicillin-streptomycin (Biowest, USA); phosphate buffered saline (PBS) (Thermo Fisher Scientific, USA); trypsin-EDTA (Thermo Fisher Scientific, USA); ezetimibe (SML1629 Sigma-Aldrich ) with ≥98% (HPLC). The cell proliferation kit I (MTT) was purchased from Roche, Switzerland with cat. no. 11465 007 001.

### Cell culture

The A375 human malignant melanoma cells, PANC-1 pancreatic cancer cells and HEK293 human embryonic kidney cells were cultured in media containing 89% DMEM, 10% FBS and 1% penicillin-streptomycin. Once confluent, the cells were washed thrice with 1X PBS and detached from T75 flasks by incubating the cells with 2 mL trypsin-EDTA for 5-10 minutes at 37 °C. 2 mL DMEM was added to the cells to stop the reaction of trypsin-EDTA.

### Protein and drug databases

The Research Collaboratory for Structural Bioinformatics (RCSB), Protein Databank (PDB) (Berman *et al*. 2000), Zinc Drug Database (Zdd) (Sterling and Irwin 2015), PubChem (Kim *et al.* 2019), and DrugBank (Wishart *et al.* 2017) databases were used. The Zdd is a database of commercially FDA approved drugs that are available worldwide as pure compounds. It constituted 2924 structures at the time of screening. The Mdm2 protein structure was downloaded from the PDB database in a pdb format (pdb id: 1ycr) and was analysed using PyMol on which the p53 peptide was removed prior to docking studies. The drug ligands were retrieved from the Zdd database in a 2D configuration. The PubChem database was utilised to obtain the structure of nutlin-3a whilst the structure of ezetimibe was obtained from the DrugBank database both in SMILE format.

### Computational 3D database screening (Pharmacophore searching)

The Schrödinger-Maestro v10.7 protein preparation wizard was used to prepare the raw PDB protein structure into a fully prepared all-atom model, and to convert 2924 2D structures into 4909 lowest energy 3D structures in a maestro format. The ionisation states of the ligands were generated at a target pH range of 7.0 ± 2 using the built-in Epik programme. The outer scoring grids were generated with different dimensions ranging from 20×20×20Ǻ to 50×50×50Ǻ in x, y, z-axis respectively to make the outer grid consistent with the shape of the protein's active site. A ligand centre box (inner grid) was generated to define the acceptable ligand centre positions during the side point search, providing a true measure of the effective search space size.

The Mdm2 p53-binding domain (Mdm2-p53BD) was used as a template on the Schrödinger's Maestro v10.7: Glide SP (Standard Precision) application to screen the Zdd database for chemical compounds. The use of Glide enabled both virtual screening and molecular docking to be conducted simultaneously taking critical residues in the Mdm2-p53BD binding pocket and the Zdd database as input. The final docking algorithm utilised is the Glide SP-algorithm, better known as the standard precision. During the docking process, the Mdm2-p53BD structure was kept rigid such that not even the hydroxyl and thiol groups could rotate, and flexibility was induced to all docking ligands. This was achieved through the ligand preparation wizard, which had generated a collection of multiple orientations of the Zdd ligand database. This generated a collection of lead compounds docked into the specified pocket with different docking scores.

### In silico pharmacokinetics

ADMET (Absorption, Distribution, Metabolism and Toxicity) studies for ezetimibe were conducted using SwissADME, ADMETSAR and ProTox II. Physicochemical properties including molecular weight (MW), molecular refractivity (MR), the count of specific atomic types and Polar surface area (PSA) were computed by employing the TPSA (Topological polar surface area) fragmental technique (which considers sulphur and phosphorus as polar atoms), lipophilicity, water solubility, pharmacokinetics and drug-likeness.

### MTT assay for cell viability

Cell viability was determined using the MTT assay, a colorimetric method that depends on mitochondrially mediated reduction of a yellow tetrazolium salt (3-(4,5-dimethylthiazol-2-yl)-2,5-diphenyltetrazolium bromide, or MTT) to the purple formazan which can be measured spectrophotometrically. The intensity of this colour is proportional to the state of metabolic activity in the cells and therefore the extent of cell viability. MTT is therefore used to measure the extent to which the drugs are toxic.

PANC-1 pancreatic cancer, A375 malignant melanoma and HEK293 human embryonic kidney cell lines were cultured to confluence in CytoOne T75 vented cell culture flasks (USA Scientific). The cells were counted using a haemocytometer and passaged/plated into CytoOne 96 well plates (USA Scientific). Approximately 5 000 cells per 200 µL were plated. The plates were incubated overnight to allow for attachment. The medium was removed after overnight incubation. The cells were then treated with 1 - 200 µM of ezetimibe for 48 hours. After incubation for 48 hours, 20 µL of MTT reagent was added and incubated for 4 hours. Approximately 200 µL of the solubilisation reagent was added to each well and incubated overnight. The MTT assay was performed using the cell proliferation kit I (MTT) (Roche, Switzerland) by following the instructions in the manual. Absorbance readings were measured at 570 nm using a 96 well plate reader. The percentage of viable cells in treated and untreated cultures was calculated and compared.

### Statistical analyses

All statistical analyses were performed using Prism GraphPad Software (San Diego, CA 92108, USA). The cell viability data was expressed as the mean ± standard deviation (SD) from three independent experiments. The IC50 values were calculated using the non-linear regression test which briefly involves conversion of the ezetimibe concentrations to log values. The log values were matched with their corresponding cell viability percentages. The one sample T-test and Wilcoxon Signed Rank Test were used to determine the statistical difference between the cell viability percentage in treated cells. The p values less than 0.05 were regarded as statistically significant.

### Results

Ezetimibe is currently used for the treatment of cholesterolaemia, is administered orally and is activated in the intestines into the pharmacologically active EZE-GLUC. The results indicate that the ezetimibe parent drug binds to Mdm2, the prototypical negative regulator of p53. Here, ezetimibe is shown to inhibit the growth of a melanoma cell line but is non-toxic to a normal human embryonic kidney cell line. This suggests that ezetimibe is a candidate drug for anti-cancer therapy when administered parenterally by injection, infusion, implantation, transdermal or other means to by-pass pre-systemic metabolism. A pharmaceutical composition comprising ezetimibe for oral administration in combination with the drug curcumin may prevent ezetimibe's pre-systemic clearance and increase its bioavailability and access for treatment of colon tumours. The pharmaceutical composition can include one or more pharmaceutically acceptable excipients, carriers or diluents.

### Molecular docking of ezetimibe to the Mdm2-p53 binding domain

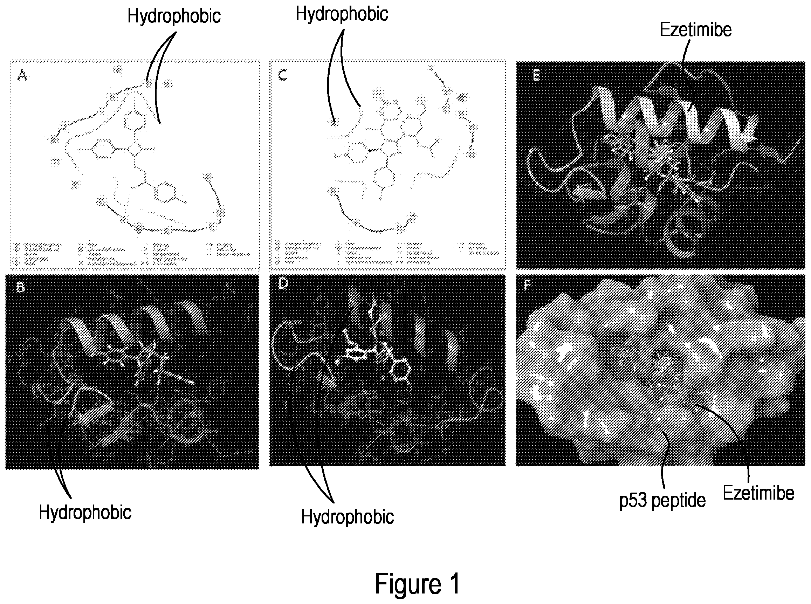

The molecular docking studies show that ezetimibe binds to the same binding pocket on Mdm2 as nutlins. Ezetimibe belongs to the azetidinone class of compounds which are characterized by a β-lactam ring (Fig 1A). Nutlins are spirooxindole compounds which were developed as non-peptidic mimics of the p53 peptide which interacts with Mdm2 (Fig 1B). Based on *in silico* modelling, it appears that ezetimibe binds snugly into the same binding site as the spirooxindole-derived nutlin3a which is in clinical trials for the treatment of cancer by reactivating p53. The nutlins were modelled on the Mdm2-binding peptide of p53 and were thus developed as non-peptidic mimics of the p53 peptide.

There are three hydrophobic pockets, Phe19, Trp23, and Leu26, based on the interacting residues on the p53-peptide, that primarily form the main anchor of the p53-Mdm2 interaction. The three ezetimibe phenyl rings fit into these three binding pockets. It is interesting that ezetimibe forms a hydrogen bond with Val93 of Mdm2 (Fig 1A and B) because Trp23 in the p53 peptide also forms a hydrogen bond with this residue and this interaction is considered to be crucial for p53-Mdm2 binding (Zhao *et al*. 2015). The binding pocket induced by ezetimibe is much smaller compared to the bulky nutlin3a-induced pocket. Moreover, superimposition of ezetimibe to the p53 peptide in the Mdm2 binding site shows a good alignment indicating that ezetimibe can act as a competitive inhibitor for p53 (Fig 1 E and F). Importantly, ezetimibe appears to fit well in the three-binding pocket of the p53 peptide with the glucuronidation site in the Phe19 pocket and the hydrogen bond in the Trp23 pocket. The halogen bond is in the Leu26 pocket. This topological arrangement would be sterically bulky for the glucuronidated version.

### Pharmacodynamics and pharmacokinetic properties of ezetimibe, nutlins and curcumin

An *in silico* study of ezetimibe ADMET was conducted as described above and was compared with known pharmacokinetics of nutlin3a (Table 1) to evaluate the drug-likeness of ezetimibe. Ezetimibe has a lower molecular weight of 409,433 g/mol compared to nutlin3a (581,494g/mol). Ezetimibe has a single rule of five violation whereas nutlin3a has four. Ezetimibe and nutlin3a have comparable lipophilicity with cLogP values of 4.33 and 4.56 respectively and are likely to be well absorbed into the systemic circulation. However, due to pre-systemic metabolism they have poor bioavailability. According to Lipinski's rule of 5, for oral administration a drug should have a LogP value less than 5.

In order to critically evaluate the mechanism of action and therapeutic potential of an Mdm2 inhibitor, it should have the following desirable properties: (a) a high binding affinity and specificity (b) potent cellular activity in cancer cells with wild-type p53, and (c) a highly desirable pharmacokinetic profile (Shangary and Wang 2009). Curcumin pharmacokinetics have been studied already with no toxicity reported in animal models and few adverse effects reported in humans. However, this could be due to the reported poor bioavailability of curcumin. According to Vareed *et al.* (2008), orally administered curcumin is absorbed and can be detected as glucuronide and sulphate conjugates in human plasma and gram doses are required to obtain detectable levels in the blood.

**Table 1: A comparative view of molecular and in silico ADME profiles of Ezetimibe and nutlin-3a**

| **Properties** | **Ezetimibe** | **Nutlins (nutlin-3a)** |
|---|---|---|
| IUPAC name | (3R,4S)-1-(4-fluorophenyl)-3-[(3S)-3-(4-fluorophenyl)-3-hydroxypropyl-4-(4-hydroxyphenyl) azetidin-2-one | 4-(4,5-bis(4-chlorophenyl)-2-(2-isopropoxy-4-methoxyphenyl)-4,5-dihydro-1H-imidazole-1-carbonyl) piperazin-2-one |
| Estimated free energy of binding, kcal/mol (Patch Dock) | | -7.7+/-0.6 kcal/mol (Warner *et al.* 2012) |
| Estimated inhibition constant, Ki (µM) | | 0.036 µM (Shangary *et al.* 2008) |
| Final intermolecular energy, kcal/mol | Not applicable | Not applicable |
| Ligand efficiency | | 9x10⁻⁴ µM |
| Torsional free energy, kcal/mol | Not applicable | Not applicable |
| Molecular weight | 409,433g/mol | 581,494 g/mol |
| Hydrogen bond acceptor | 5 | 5 |
| Hydrogen bond donor | 2 | 1 |
| Rotatable bonds | 6 | 8 |
| Rule of five (No. of violations) | 1 | 4 |
| ClogP | 4.33 | 4.56 |
| Solubility (SILICOS-IT) | 2.55×10⁻⁵mg/ml | 4.12×10⁻⁷mg/ml |
| Blood-brain barrier (ADMETSAR probability) | 0.9074 | 0.7397 |
| Human intestinal absorption (ADMETSAR probability) | 0.9899 | 1.0000 |
| Carcinogens (ADMETSAR probability) | 0.8328 | 0.6361 |
| Acute oral toxicity (ADMETSAR probability) | 0.5892 | 0.6605 |
| Aqueous solubility (logS) (ADMETSAR) | -3.8761 | -3.2817 |
| Rat acute toxicity (LDso, mol/kg) (ADMETSAR) | 2.4979 | 2.5907 |
| Solvent accessibility or polar surface area (Å²) | 60.77Å² | 83.47Å² |
| Drug score (docking score) | -7.885 | |
| Binding residues (Mdm2 p53BD) | VAL93, LYS94, GLN72, GLY58, PHE86, ILE103, LEU82, PHE91, LEU57, LEU54, ILE99, TYR100, TYR67, MET62, ILE61 | VAL93, GLN72, GLY58, LEU54 ILE99, TYR100, MET62, ILE61 |

**Table 2: Pharmacodynamics and pharmacokinetics comparison of ezetimibe and curcumin**

| | **Ezetimibe** | **Curcumin** | **Reference** |
|---|---|---|---|
| Target | Nieman-Pick C1 Like 1 | Multiple | (Reddy and Aggarwal 1994; Davis and Veltri 2007) |
| Action | Prevents cholesterol absorption in the jejunal brush border | General kinase inhibitor | (Reddy and Aggarwal 1994; Kosoglou *et al.* 2005) |
| Active form | Ezetimibe glucuronide | Curcumin | (Reddy and Aggarwal 1994; Davis and Veltri 2007) |
| Metabolism | UGT1A1, UGT1A3 and UGT2B15 | | (Davis and Veltri 2007; Berginc *et al.* 2012) |
| Aqueous Solubility | Poor | Poor | (Kosoglou *et al.* 2005; Berginc *et al.* 2012) |
| Bioavailability | Almost negligible | Poor | (Kosoglou *et al.* 2005; Vareed *et al*. 2008) |

### Drug toxicity

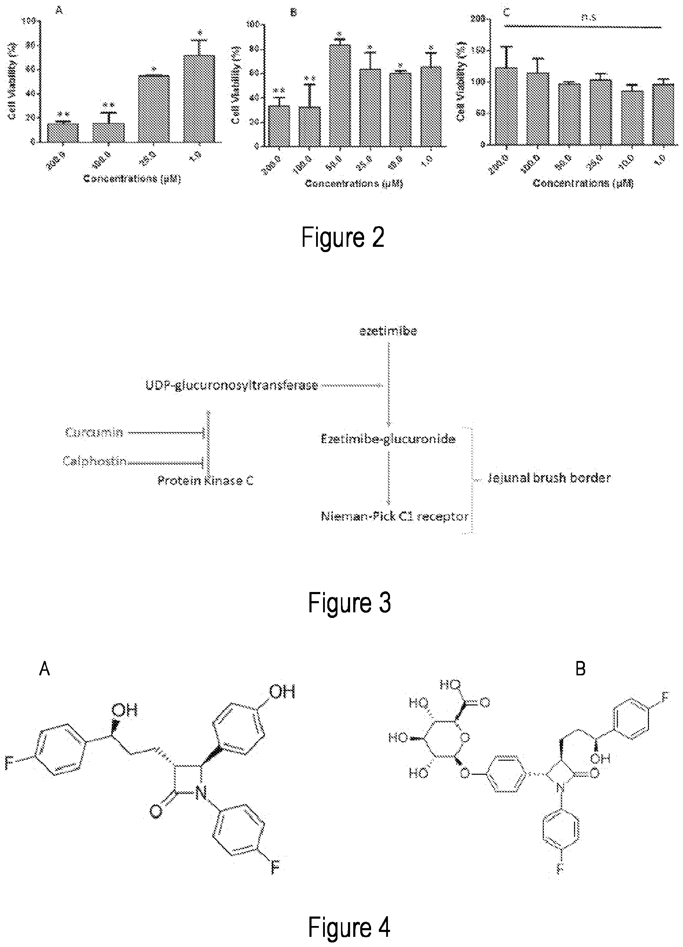

The toxicity of ezetimibe was tested in the A375 human melanoma cell line (Fig 2A), the PANC-1 human pancreatic cell line (Fig 4B) and the HEK293 normal human embryonic kidney cell line (Fig 4C) using the MTT assay. Ezetimibe strongly inhibits the growth of the melanoma cell line with a calculated IC₅₀ of 30.71 µM using the GraphPad statistical analysis software v.5.1 (nonlinear regression (curve fit)), and to a lesser extent the pancreatic cancer cell line as well. An IC₅₀ value of 71.58 µM was calculated for the pancreatic cancer cells. At these concentrations of 0.1-200 µM, ezetimibe does not exhibit toxicity to the normal human embryonic kidney cell line. Concentrations between 20-80 µM of ezetimibe were toxic to cancer cells. Interestingly, several studies show that Mdm2 and Mdm4 are highly expressed in melanoma (Muthusamy *et al.* 2006; Gembarska *et al.* 2012). Ezetimibe inhibits approximately 20% of the melanoma cell line at the lowest concentrations tested (1µM) and more than 80% at the highest concentration (200 µM).

### Discussion

The *in silico* molecular docking studies indicate that ezetimibe binds snugly into the p53-binding site of Mdm2. It is clear that EZE-GLUC would not fit into the same binding site. Nutlin3a which binds to the same site has a bulkier molecular structure and higher molecular weight making ezetimibe a better candidate with good drug-likeness. Hence, the pharmacokinetic parameters that were predicted using the *in silico* approach support this view. The toxicity assay using the A375 melanoma cell line shows cytotoxicity at a micromolar concentration with an IC₅₀ of 30.7 µM. This IC₅₀ is rather high and therefore introduces some doubts about the specificity of the inhibition. It is interesting that clear toxicity is shown on the melanoma cell compared to the pancreatic and normal cell lines, because Mdm2 is known to be overexpressed in melanoma. Ezetimibe may therefore be effective in treating cancers, including cancers selected from breast cancer, cervical cancer, colorectal cancer, fallopian tube carcinoma, gastric cancer, glioma, Hodgkin's lymphoma, leukemia, liver cancer, lung cancer, melanoma, non-Hodgkin's lymphoma, oral cancer, ovarian cancer, pancreatic cancer, renal cell carcinoma, thyroid cancer, and other cancers.

### Pharmaceutical composition for oral administration of ezetimibe for treatment of colon cancer

Currently, ezetimibe is administered orally so that its EZE-GLUC metabolite can block intestinal absorption of cholesterol from food by binding to the NPC1L1 receptor. This blocking mechanism is not well accomplished by the ezetimibe parent drug as it has to be converted into its active form (EZE-GLUC) by UDP-glucuronosyltransferases (UGTs) that are present in the intestines. For glucuronidation of ezetimibe to occur, the UDP-glucuronosyltransferase has to be phosphorylated by a kinase. Since curcumin is a potent reversible inhibitor of kinases, it will inhibit the conversion of ezetimibe thereby allowing it to be available to cancerous cells in the colon. Orally administered curcumin can therefore be used for reversible inhibition of UGTs in the small intestine and the inhibition of glucuronidation (*Basu et al.* 2007). Curcumin can therefore be combined with ezetimibe in a pharmaceutical composition, with a pharmaceutically acceptable excipient, carrier or diluent to target colon cancer. A schematic of these molecular interactions is represented by **Figure 3****.**

A further advantage of this combination is that it could also be used to treat other cancers as the parent drug is absorbed into the circulatory system and reaches other parts of the body, such as breast cancer, cervical cancer, colorectal cancer, fallopian tube carcinoma, gastric cancer, glioma, Hodgkin's lymphoma, leukemia, liver cancer, lung cancer, melanoma, non-Hodgkin's lymphoma, oral cancer, ovarian cancer, pancreatic cancer, renal cell carcinoma, thyroid cancer, and others.

The key limitation is that both ezetimibe and curcumin are highly insoluble and have low bioavailability (Berginc *et al.* 2012). This can be addressed by the formulation of the pharmaceutical composition for different cancers.

### Discussion

It is believed that the original form of ezetimibe binds to Mdm2 which is crucial in carcinogenesis as an oncogene as well as a negative regulator of the tumour suppressor, p53. Ezetimibe probably binds to the p53-binding domain of Mdm2 (Fig 3) and may therefore inhibit carcinogenesis.

In contrast to its current use to reduce cholesterol levels, ezetimibe must be in its original form for the treatment of cancer. The present invention provides a pharmaceutical composition comprising ezetimibe in combination with curcumin for use in the treatment of colon cancer, avoiding the metabolism of ezetimibe to ezetimibe glucuronide. The present invention also provides for a pharmaceutical composition comprising ezetimibe in combination with curcumin for use in the treatment of other solid tumours, including breast cancer, cervical cancer, colorectal cancer, fallopian tube carcinoma, gastric cancer, glioma, Hodgkin's lymphoma, leukemia, liver cancer, lung cancer, melanoma, non-Hodgkin's lymphoma, oral cancer, ovarian cancer, pancreatic cancer, renal cell carcinoma, thyroid cancer, and others.

### References

Basu NK, Kole L, Basu M, McDonagh AF and Owens IS 2007. Targeted inhibition of glucuronidation markedly improves drug efficacy in mice - a model. Biochem Biophys Res Commun 360: 7-13.
Berginc K, Trontelj J, Basnet NS and Krist! A 2012. Physiological barriers to the oral delivery of curcumin. Die Pharmazie - An International Journal of Pharmaceutical Sciences 67: 518-524.
Berman HM, Westbrook J, Feng Z, Gilliland G, Bhat TN, Weissig H, Shindyalov IN and Bourne PE 2000. The Protein Data Bank Nucleic Acids Res 28: 235-242.
Davis HR and Veltri EP 2007. Zetia: Inhibition of Niemann-Pick C1 Like 1 (NPC1L1) to Reduce Intestinal Cholesterol Absorption and Treat Hyperlipidemia. Journal of Atherosclerosis and Thrombosis 14: 99-108.
Gembarska A, Luciani F, Fedele C, Russell EA, Dewaele M, Villar S, Zwolinska A, Haupt S, de Lange J, Yip D et al. 2012. MDM4 is a key therapeutic target in cutaneous melanoma. Not Med 18: 1239.
Kim S, Chen J, Cheng T, Gindulyte A, He J, He S, Li Q, Shoemaker BA, Thiessen PA, Yu B et al. 2019. PubChem 2019 update: improved access to chemical data. Nucleic Acids Res 47.
Kosoglou T, Statkevich P, Johnson-Levonas A, Paolini JF, Bergman AJ and Alton KB. 2005. Ezetimibe. Clin Pharmacokinet 44: 467-494.
Muthusamy V, Hobbs C, Nogueira C, Cordon-Cardo C, McKee PH, Chin L and Bosenberg MW 2006. Amplification of CDK4 and MDM2 in malignant melanoma. Genes Chromosomes Cancer 45: 447-454.
Reddy S and Aggarwal BB 1994. Curcumin is a non-competitive and selective inhibitor of phosphorylase kinase. FEBS Lett 341: 19-22.
Shangary S and Wang S 2009. Small-Molecule Inhibitors of the MDM2-p53 Protein-Protein Interaction to Reactivate p53 Function: A Novel Approach for Cancer Therapy. Annu Rev Pharmacol Toxicol 49: 223-241.
Shangary S, Ding K, Qiu S, Nikolovska-Coleska Z, Bauer JA, Liu M, Wang G, Lu Y, McEachern D, Bernard D, Bradford CR, Carey TE and Wang S 2008. Reactivation of p53 by a specific MDM2 antagonist (MI-43) leads to p21-mediated cell cycle arrest and selective cell death in colon cancer. Mol Cancer Ther 7(6): 1533-1542.
Sterling T and Irwin JJ 2015. ZINC 15 - Ligand discovery for everyone. J Chem Inf Model 55: 2324-2337.
Vareed SK, Kakarala M, Ruffin MT, Crowell JA, Normolle DP, Djuric Z and Brenner DE 2008. Pharmacokinetics of curcumin conjugate metabolites in healthy human subjects. Cancer epidemiology, biomarkers & prevention: a publication of the American Association for Cancer Research, cosponsored by the American Society of Preventive Oncology 17: 1411-1417.
Warner WA, Sanchez R, Dawoodian A, Li E and Momand J 2012. Identification of FDA-approved drugs that computationally bind to MDM2. Chem Biol Drugs Des 80(4): 631-637
Wishart DS, Feunang YD, Guo AC, Lo EJ, Marcu A, Grant JR, Sajed T, Johnson D, Li C, Sayeeda Z et al. 2017. DrugBank 5.0: a major update to the DrugBank database for 2018. Nucleic Acids Res. 46(D1): D1074-D1082
Zhao Y, Aguilar A, Bernard D and Wang S 2015. Small-molecule inhibitors of the MDM2-p53 protein-protein interaction (MDM2 Inhibitors) in clinical trials for cancer treatment. J Med Chem 58: 1038-1052.

## Claims

1. A pharmaceutical composition comprising ezetimibe, or a pharmaceutically acceptable salt thereof, and curcumin for use in a method of treating a cancer in a patient in need thereof, wherein the cancer is **characterized by** overexpression of Mdm2.

2. The pharmaceutical composition for use according to claim 1, wherein the cancer is a solid cancer, such as colon cancer, or a melanoma.

3. The pharmaceutical composition for use according to claim 1 or 2, wherein the pharmaceutical composition is formulated for systemic or oral administration to the patient.

4. The pharmaceutical composition for use according to any one of claims 1 to 3, wherein the pharmaceutical composition is in the form of a formulation, an injection, a tablet, or a capsule.

5. A pharmaceutical composition comprising ezetimibe, or a pharmaceutically acceptable salt thereof, for use in a method of treating a cancer in a patient in need thereof, wherein the pharmaceutical composition is for administration with curcumin and wherein the cancer is **characterized by** overexpression of Mdm2.

6. The pharmaceutical composition for use according to claim 5, wherein the cancer is a solid cancer, including colon cancer, or a melanoma.

7. The pharmaceutical composition for use according to claim 5 or 6, wherein the pharmaceutical composition is formulated for systemic or oral administration to the patient.

8. The pharmaceutical composition for use according to any one of claims 5 to 7, wherein the pharmaceutical composition is in the form of a formulation, an injection, a tablet, or a capsule.

## Patentansprüche

1. Eine pharmazeutische Zusammensetzung, umfassend Ezetimib oder ein pharmazeutisch akzeptables Salz davon und Curcumin zur Verwendung in einem Verfahren zur Behandlung von Krebs bei einem Patienten, wobei der Krebs durch eine Überexpression von Mdm2 gekennzeichnet ist.

2. Die pharmazeutische Zusammensetzung zur Verwendung nach Anspruch 1, wobei der Krebs ein solider Krebs, wie z. B. Dickdarmkrebs, oder ein Melanom ist.

3. Die pharmazeutische Zusammensetzung zur Verwendung nach Anspruch 1 oder 2, wobei die pharmazeutische Zusammensetzung zur systemischen oder oralen Verabreichung an den Patienten formuliert ist.

4. Die pharmazeutische Zusammensetzung zur Verwendung nach einem der Ansprüche 1 bis 3, wobei die pharmazeutische Zusammensetzung in Form einer Formulierung, einer Injektion, einer Tablette oder einer Kapsel vorliegt.

5. Eine pharmazeutische Zusammensetzung, umfassend Ezetimib oder ein pharmazeutisch akzeptables Salz davon, zur Verwendung in einem Verfahren zur Behandlung von Krebs bei einem Patienten, wobei die pharmazeutische Zusammensetzung zur Verabreichung mit Curcumin bestimmt ist und wobei der Krebs durch eine Überexpression von Mdm2 gekennzeichnet ist.

6. Die pharmazeutische Zusammensetzung zur Verwendung gemäß Anspruch 5, wobei der Krebs ein solider Krebs, einschließlich Dickdarmkrebs, oder ein Melanom ist.

7. Die pharmazeutische Zusammensetzung zur Verwendung nach Anspruch 5 oder 6, wobei die pharmazeutische Zusammensetzung zur systemischen oder oralen Verabreichung an den Patienten formuliert ist.

8. Die pharmazeutische Zusammensetzung zur Verwendung nach einem der Ansprüche 5 bis 7, wobei die pharmazeutische Zusammensetzung in Form einer Formulierung, einer Injektion, einer Tablette oder einer Kapsel vorliegt.

## Revendications

1. Composition pharmaceutique comprenant de l'ézétimibe, ou sel pharmaceutiquement acceptable de celle-ci, et de la curcumine pour une utilisation dans un procédé de traitement d'un cancer chez un patient ayant besoin de celui-ci, dans laquelle le cancer est **caractérisé par** une surexpression de Mdm2.

2. Composition pharmaceutique pour une utilisation selon la revendication 1, dans laquelle le cancer est un cancer solide, tel que le cancer du côlon, ou un mélanome.

3. Composition pharmaceutique pour une utilisation selon la revendication 1 ou 2, dans laquelle la composition pharmaceutique est formulée pour une administration systémique ou orale au patient.

4. Composition pharmaceutique pour une utilisation selon l'une quelconque des revendications 1 à 3, dans laquelle la composition pharmaceutique est dans la forme d'une formulation, une injection, un comprimé, ou une gélule.

5. Composition pharmaceutique comprenant de l'ézétimibe, ou sel pharmaceutiquement acceptable de celle-ci, pour une utilisation dans un procédé de traitement d'un cancer chez un patient ayant besoin de celui-ci, dans laquelle la composition pharmaceutique est destinée à une administration avec de la curcumine et dans laquelle le cancer est **caractérisé par** une surexpression de Mdm2.

6. Composition pharmaceutique pour une utilisation selon la revendication 5, dans laquelle le cancer est un cancer solide, incluant le cancer du côlon, ou un mélanome.

7. Composition pharmaceutique pour une utilisation selon la revendication 5 ou 6, dans laquelle la composition pharmaceutique est formulée pour une administration systémique ou orale au patient.

8. Composition pharmaceutique pour une utilisation selon l'une quelconque des revendications 5 à 7, dans laquelle la composition pharmaceutique est dans la forme d'une formulation, une injection, un comprimé, ou une gélule.
